(19) Europäisches Patentamt
European Patent Office
Office européen des brevets

(11) Veröffentlichungsnummer: **0 028 730**
**B1**

(12) # EUROPÄISCHE PATENTSCHRIFT

(45) Veröffentlichungstag der Patentschrift: **26.05.82**

(51) Int. Cl.³: **C 07 C 143/60**, C 07 C 139/00

(21) Anmeldenummer: **80106376.9**

(22) Anmeldetag: **20.10.80**

(54) **Verfahren zur Herstellung von 1,6-Diaminonaphthalin-4,8-disulfonsäure.**

(30) Priorität: **31.10.79 DE 2944094**

(43) Veröffentlichungstag der Anmeldung: **20.05.81 Patentblatt 81/20**

(45) Bekanntmachung des Hinweises auf die Patenterteilung: **26.05.82 Patentblatt 82/21**

(84) Benannte Vertragsstaaten: **CH DE FR GB IT LI**

(56) Entgegenhaltungen:
**DE-B2-2 265 291**

**BEILSTEINS HANDBUCH DER ORGANISCHEN CHEMIE
4. Auflage, Band 14, 1931
VERLAG JULIUS SPRINGER, Berlin
Seite 787, Zeilen 37 bis 44**

**HOUBEN-WEYL
«Methoden der Organischen Chemie»
4. Auflage, Band 11/1, 1957
GEORG THIEME VERLAG, Stuttgart
Seite 160**

(73) Patentinhaber: **BAYER AG, Zentralbereich Patente, Marken und Lizenzen, D-5090 Leverkusen 1, Bayerwerk (DE)**

(72) Erfinder: **Kramer, Horst-Dieter, Dr., Gabriele-Münter-Strasse 6, D-5090 Leverkusen 1 (DE)**
Erfinder: **Potthast, Ruthard, Dr., Martin-Buber-Strasse 45, D-5090 Leverkusen 3 (DE)**
Erfinder: **Krüger, Bruno, Dr., Haferkamp 1, D-5000 Köln 80 (DE)**

**EP 0 028 730 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von 1,6-Diaminonaphthalin-4,8-disulfonsäure

Die vorliegende Erfindung betrifft ein Verfahren zur Herstellung von 1,6-Diaminonaphthalin-4,8-disulfonsäure aus 1-Amino-6-naphthol-4,8-disulfonsäure durch Aminierung mit wässriger Ammoniaklösung bei erhöhten Temperaturen und erhöhtem Druck. 1,6-Diaminonaphthalin-4,8-disulfonsäure ist eine wichtige Kupplungskomponente für die Herstellung von Azofarbstoffen.

Aus DRP 72665 ist bekannt, dass man 1,6-Diaminonaphthalin-4,8-disulfonsäure in folgender Weise herstellen kann:

Naphthalin wird bei niedriger Temperatur zur Naphthalin-1,5-disulfonsäure (Armstrongsäure) disulfiert und ohne Zwischenisolierung zur 1,6-Dinitronaphthalin-4,8-disulfonsäure dinitriert. Die anschliessende Reduktion führt zur 1,6-Diaminonaphthalin-4,8-disulfonsäure, die durch Ansäuern der Reduktionsbrühen isoliert wird.

Bei diesem Verfahren ist nachteilig, dass erhebliche Ausbeuteeinbussen durch die während der Nitrierungsreaktion parallel ablaufenden Oxidationsreaktionen verursacht werden, so dass die Gesamtausbeute an 1,6-Diaminonaphthalin-4,8-disulfonsäure, bezogen auf Naphthalin, nicht über 25% d.Th. beträgt. Eine hohe Belastung der Prozessabwässer mit organischer Substanz ist die Folge.

Ein weiterer Nachteil des geschilderten Verfahrens besteht in der schwer beherrschbaren Reaktionsführung der Nitrierungsreaktion besonders bei der Weiternitrierung der intermediär entstandenen 2-Nitro-naphthalin-4,8-disulfonsäure.

Es wurde nun ein Verfahren zur Herstellung von 1,6-Diaminonaphthalin-4,8-disulfonsäure gefunden, dadurch gekennzeichnet, dass man von 1-Amino-6-naphthol-4,8-disulfonsäure ausgeht und diese mit wässriger Ammoniaklösung bei erhöhtem Druck und erhöhten Temperaturen umsetzt.

Das in das erfindungsgemässe Verfahren eingesetzte Produkt fällt als bisher technisch nicht genutztes Nebenprodukt bei der Verschmelzung der 1-Naphthyl-amin-4,6,8-trisulfonsäure zu 1-Amino-8-naphthol-4,6-disulfonsäure (K-Säure) an. Dabei kann die Herstellung der 1-Amino-8-naphthol-4,6-disulfonsäure z.B. erfolgen nach Ullmann, Enzyklopädie der technischen Chemie, 3. Auflage, Band 12, Seite 622.

Die 1-Amino-6-naphthol-4,8-disulfonsäure kann als wässrige Lösung mit einem Gehalt von 20-50 Gew.-%, gerechnet als freie Säure mit dem Molekulargewicht 319 oder als Feuchtprodukt mit einem Festgehalt von 50-85 Gew.-%, vorzugsweise 70-85 Gew.-% eingesetzt werden.

Die im erfindungsgemässen Verfahren eingesetzten wässrigen Ammoniaklösungen werden vorzugsweise im Überschuss verwendet. Pro Mol 1-Amino-6-naphthol-4,8-disulfonsäure können beispielsweise 2-40 Mol Ammoniak eingesetzt werden. Besonders bevorzugt ist der Einsatz von 5-12 Mol Ammoniak auf 1 Mol 1-Amino-6-naphthol-4,8-disulfonsäure.

Die Umsetzung kann bei Temperaturen zwischen 120 und 200°C in einem geschlossenen Gefäss durchgeführt werden. Für das erfindungsgemässe Verfahren finden vorzugsweise Drücke im Bereiche von 10 bis 30 bar Anwendung. Die Reaktionszeit wird bestimmt von der Reaktionstemperatur sowie der angewandten Konzentration des Ammoniaks.

Das erfindungsgemässe Verfahren vermeidet die geschilderten Nachteile des bekannten Verfahrens, da es von einer Hydroxynaphthylamindisulfonsäure geeigneter Konstitution ausgeht und durch einfache Amidierung mit wässrigem Ammoniak die 1,6-Diaminonaphthalin-4,8-disulfonsäure ergibt.

Das Verfahren wird durch folgendes Reaktionsschema formelmässig erläutert:

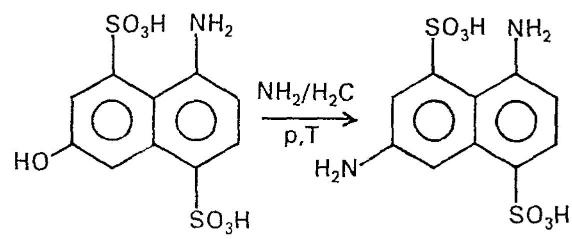


Das erfindungsgemässe Verfahren bietet gegenüber den bisher üblichen Verfahren der Dinitrierung der Naphthalin-1,5-disulfonsäure erhebliche Vorteile, da ein bisher nicht genutztes bei der Herstellung der 1-Amino-8-naphthol-4,8-disulfonsäure in Mengen von 8-12% anfallendes Nebenprodukt technisch verwertet wird. Damit verbunden ist eine Verringerung der Abwasserbeladung beim Verfahren zur Herstellung der 1-Amino-8-naphthol-4,8-disulfonsäure (K-Säure).

Beispiel 1

In einem 2,5 Liter V4A-Autoklav werden 319 g 1-Amino-6-naphthol-4,8-disulfonsäure (MG 319) als trockene 100%ige Ware mit 1200 ml einer wässrigen Ammoniaklösung (Gehalt 170 g $NH_3$ 100%) 20 Stunden auf einer Temperatur von 185°C gehalten. Nach dem Erkalten des Reaktionsgemisches füllt man mit Wasser auf ein Volumen von 2000 ml auf, fügt 50 ml mit einer 50%igen Natronlauge hinzu, erhitzt die Lösung und treibt das überschüssige Ammoniak ab. Die dunkle Lösung wird zur Klärung über eine Nutsche gegeben und das Filtrat bei 90°C mit konzentrierter Salzsäure bis zu einem pH von 1,5 angesäuert.

Die erhaltene hellbraune Suspension wird 45 Minuten bei 90° gerührt und Schwefeldioxid abgetrieben. Die Suspension wird mit Wasser auf ein Volumen von 3000 ml aufgefüllt und allmählich abgekühlt. Bei 20°C saugt man den Niederschlag ab und wäscht mit 500 ml kalten Wasser.

Man erhält 245 g einer feuchten, hellbraunen Paste mit einem Feststoffgehalt von 79%.

Die Ausbeute beträgt 194 g (61% der Theorie) 1,6-Diaminonaphthalin-4,8-disulfonsäure.

Beispiel 2

1221 g (3,8 Mol) 1-Amino-6-naphthol-4,8-disulfonsäure werden mit 1500 ml Wasser und 1076 ml (646 g entsprechend 38 Mol) Ammoniak 100% in einem 5 Liter-Stahl-Autoklaven 30 Stunden auf 165°C gehalten. Nach der Reaktion füllt man mit Wasser auf ein Gesamtvolumen von 6000 ml an. Der pH-Wert der Lösung beträgt 10,4.

Durch Zugabe von 283 ml 50%iger Natronlauge stellt man auf einen pH von 11,2. Man treibt den überschüssigen Ammoniak ab und gibt die Lösung zur Klärung über eine Nutsche, deren Boden mit A-Kohle belegt ist. Zur Isolierung des Reaktionsproduktes trägt man die Lösung in 3000 ml Wasser ein und versetzt innerhalb von 45 Minuten bei 90°C mit 780 ml konzentrierter Salzsäure bis auf pH 1,5. Man rührt die erhaltene gelbbraune Suspension bis zur völligen Entfernung des Schwefeldioxids. Anschliessend wird auf ein Volumen von 10 l aufgefüllt und über 24 Stunden allmählich abgekühlt.

Der sehr feinkörnige Niederschlag wird bei Raumtemperatur abgesaugt und mit 1000 ml kaltem Wasser gewaschen. Die erhaltene Paste ist hellgelb.

Die Ausbeute beträgt 1470 g feuchtes Produkt mit einem Feststoffgehalt von 54,1% entsprechend 796 g 100% (65,2% der Theorie).

Beispiel 3

1185 g 1-Amino-6-naphthol-4,8-disulfonsäure trocken mit einem Feststoffgehalt von 86,2% entsprechend 1021 g 100% (3,2 Mol) werden mit 544 g Ammoniak 100% = 32 Mol) und 2176 ml Wasser 17 Stunden lang bei 185°C in einem 5 Liter V4A-Autoklav erhitzt. Der Maximaldruck beträgt 26 bar. Die noch heisse Reaktionslösung wird in ein 5 Liter Becherglas gespült.

Anschliessend lässt man die dunkle Lösung zusammen mit 2800 ml 48%iger Schwefelsäure bei 90-95°C in ein 10 Liter Becherglas laufen. Die Suspension wird so lange gerührt, bis kein Schwefeldioxid mehr entweicht und dann allmählich abkühlt. Bei 40°C wird der Niederschlag abgesaugt und mit 1000 ml warmen Wasser gewaschen.

Man erhält die 1,6-Diaminonaphthalin-4,8-disulfonsäure als feuchte Paste. Ausbeute 1230 g Feuchtprodukt mit einem Feststoffgehalt von 51,2% entsprechend 630 g 100% als dunkelbraunes Pulver (61,7% der Theorie).

Die 1,6-Diaminonaphthalin-4,8-disulfonsäure enthält nach der Analyse folgende Verunreinigungen:

1,5% 2,6-Diaminonaphthalin-4,8-disulfonsäure sowie max.

1,2% unbekannte Stoffe.

## Patentansprüche

1. Verfahren zur Herstellung von 1,6-Diaminonaphthalin-4,8-disulfonsäure, dadurch gekennzeichnet, dass man 1-Amino-6-naphthol-4,8-disulfonsäure mit wässriger Ammoniaklösung bei erhöhtem Druck und erhöhter Temperatur umsetzt.

2. Verfahren gemäss Anspruch 1, dadurch gekennzeichnet, dass man die Umsetzung bei Temperaturen zwischen 120 und 200°C und bei Drükken zwischen 10 bis 30 bar durchführt.

## Claims

1. Process for the preparation of 1,6-diaminonaphthalene-4,8-disulphonic acid, characterised in that 1-amino-6-naphthol-4,8-disulphonic acid is reacted with aqueous ammonia solution under increased pressure and at elevated temperature.

2. Process according to Claim 1, characterised in that the reaction is carried out at temperatures between 120 and 200°C and under pressures between 10 and 30 bars.

## Revendications

1. Procédé de préparation de l'acide 1,6-diaminonaphtalène-4,8-disulfonique, caractérisé en ce que l'on fait réagir l'acide 1-amino-6-naphtol-4,8-disulfonique avec l'ammoniaque à température et pression élevées.

2. Procédé selon la revendication 1, caractérisé en ce que la réaction est effectuée à des températures de 120 à 200°C et des pressions de 10 à 30 bars.